# EUROPEAN PATENT APPLICATION

(11) **EP 2 210 563 A1**
(43) Date of publication of application: **28.07.2010**
(21) Application number: 10250089.9
(22) Date of filing: 20.01.2010
(51) Int. Cl.: A61B 8/08, A61B 5/06

(54) **Catheter with isolation between ultrasound transducer and position sensor**

(30) Priority: 23.01.2009 US 358745
(71) Applicant: Biosense Webster, Diamond Bar, CA 91765 (US)
(72) Inventor: Lichtenstein, Yoav, Raanana 43727 (IL)
(74) Representative: Small, Gary James

(57) **Abstract**

Medical apparatus includes an invasive probe, for insertion into a body of a living subject. An ultrasonic subsystem includes an ultrasonic transducer contained in the probe and image processing circuitry, which is located outside the probe and is coupled to communicate with the ultrasonic transducer in the probe. A position sensing subsystem includes a position transducer contained in the probe and position tracking circuitry, which is located outside the probe and is coupled to communicate with the position transducer so as to determine position coordinates of the ultrasonic transducer in the body. The position sensing subsystem is electrically isolated from the ultrasonic subsystem.

## Description

### FIELD OF THE INVENTION

The present invention relates generally to invasive medical devices, and specifically to invasive probes that combine imaging and position sensing functions.

### BACKGROUND OF THE INVENTION

A catheter with acoustic transducers may be used for non-contact imaging of the endocardium. For example, U.S. Patents 6,716,166 and 6,773,402, whose disclosures are incorporated herein by reference, describe a system for 3-D mapping and geometrical reconstruction of body cavities, particularly of the heart. The system uses a cardiac catheter comprising a plurality of acoustic transducers. The transducers emit ultrasonic waves that are reflected from the surface of the cavity and are received again by the transducers. The distance from each of the transducers to a point or area on the surface opposite the transducer is determined, and the distance measurements are combined to reconstruct the 3-D shape of the surface. The catheter also comprises position sensors, which are used to determine location and orientation coordinates of the catheter within the heart.

U.S. Patent Application Publication 2006/0241445, whose disclosure is incorporated herein by reference, describes a method and apparatus for modeling an anatomical structure, such as a chamber of the heart. A probe that comprises an array of ultrasound transducers and a position sensor is used to image a target organ or structure in the patient's body. In one embodiment, the probe comprises a catheter, which is inserted into the patient's heart. The probe acquires multiple 2-D ultrasound images of the target organ and sends them to an image processor. For each image, location and orientation coordinates of the probe are measured using the position sensor. A three-dimensional (3-D) model of the anatomical structure is constructed, based on the ultrasound images and on the measured location and orientation coordinates.

### SUMMARY OF THE INVENTION

Combining position sensing and ultrasonic imaging functions in a single probe, such as a cardiac catheter, may give rise to safety concerns. The ultrasonic subsystem, including an ultrasonic transducer contained in the probe and external processing circuitry, may generate relatively high voltages and is typically held at ground potential and electrically isolated from the patient. On the other hand, the position sensing subsystem, including a position transducer in the probe and external position tracking circuitry, may be held at the "applied part" potential of the patient's body, which should be kept isolated from the ground for safety reasons. The position transducer and ultrasonic transducer, however, are typically located in close proximity to one another in the probe. A short circuit between these components or their associated wiring could violate the desired isolation and endanger the patient.

Embodiments of the present invention that are described hereinbelow provide secure electrical isolation between position sensing and ultrasonic subsystems in an invasive imaging system. The isolation may be achieved by using isolation circuitry to convey signals between the position sensor in the probe and the external position tracking circuitry without creating a conductive path between the position sensor and the position tracking circuitry.

There is therefore provided, in accordance with an embodiment of the present invention, medical apparatus, including:
an invasive probe, for insertion into a body of a living subject;
an ultrasonic subsystem, including an ultrasonic transducer contained in the probe and image processing circuitry, which is located outside the probe and is coupled to communicate with the ultrasonic transducer in the probe; and
a position sensing subsystem, including a position transducer contained in the probe and position tracking circuitry, which is located outside the probe and is coupled to communicate with the position transducer so as to determine position coordinates of the ultrasonic transducer in the body,
wherein the position sensing subsystem is electrically isolated from the ultrasonic subsystem.

In some embodiments, the invasive probe includes a catheter for insertion into a heart of the subject. In one embodiment, the ultrasonic transducer is configured to capture a plurality of ultrasonic input images of an organ at different, respective positions of the probe within the body, and the image processing circuitry is configured to combine the input images, using the position coordinates, to generate a three-dimensional image of the organ. Additionally or alternatively, the position sensing subsystem includes one or more magnetic field generators, which are configured to generate a magnetic field within the body, and the position transducer includes a magnetic field sensor, which is configured to output position signals to the position tracking circuitry responsively to the magnetic field.

Typically, the ultrasonic transducer is held at a ground potential by the ultrasonic subsystem, while the position sensing subsystem is at a non-ground potential.

In a disclosed embodiment, the apparatus includes isolation circuitry, which is interposed between the position transducer and the position tracking circuitry so as to convey position signals between the position transducer and the position tracking circuitry without creating a conductive path between the position transducer and the position tracking circuitry. Typically, the isolation circuitry includes one or more isolation transformers.

There is also provided, in accordance with an embodiment of the present invention, a method for producing an invasive medical system, including:
providing an invasive probe, for insertion into a body of a living subject;
assembling an ultrasonic subsystem by installing an ultrasonic transducer in the probe and coupling the ultrasonic transducer to communicate with image processing circuitry outside the probe;
assembling a position sensing subsystem by installing a position transducer in the probe and coupling the position transducer to communicate with position tracking circuitry outside the probe so as to determine position coordinates of the ultrasonic transducer in the body; and
electrically isolating the position sensing subsystem from the ultrasonic subsystem.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a system for cardiac imaging, in accordance with an embodiment of the present invention;
Fig. 2 is a block diagram showing electrical components of the system of Fig. 1, in accordance with an embodiment of the present invention; and
Fig. 3 is a block diagram showing details of isolation circuitry, in accordance with an embodiment of the present invention.

### DETAILED DESCRIPTION OF THE INVENTION

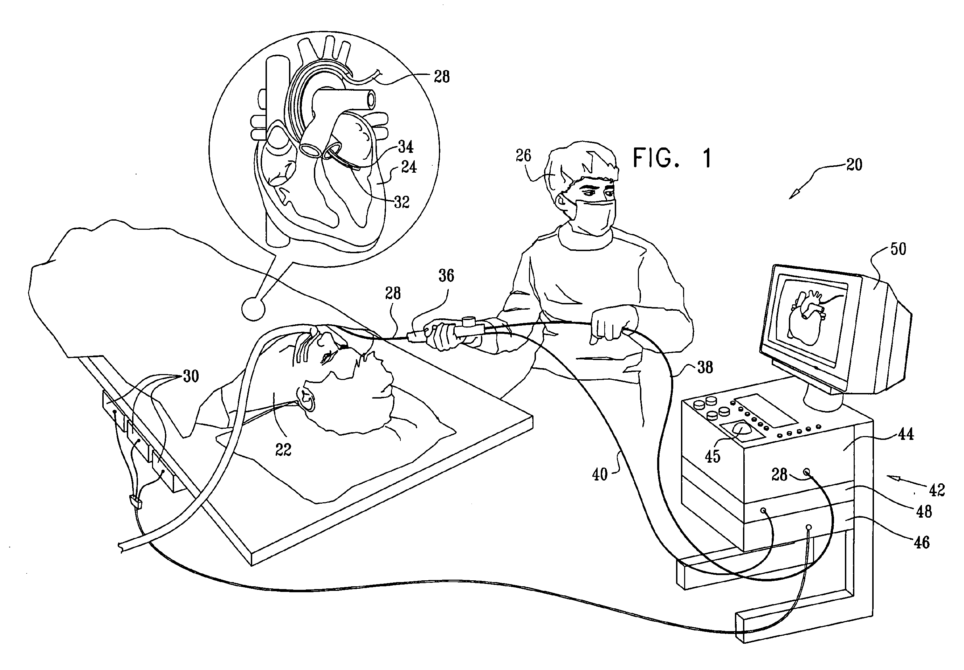

Fig. 1 is a schematic, pictorial illustration of a system 20 for imaging a heart 24 of a patient 22, in accordance with an embodiment of the present invention. The system comprises a catheter 28, which is inserted by an operator 26, such as a physician, into a chamber of the heart through a vein or artery. The operator controls and manipulates the catheter using a handle 36, which also contains certain circuitry, as described further hereinbelow. System 20 comprises a positioning subsystem that measures position (location and orientation) coordinates of catheter 28. In one embodiment, the positioning subsystem comprises a magnetic position tracking system, comprising a magnetic field generator in the form of a set of external radiator coils 30, which are located in a fixed position external to the patient. Coils 30 generate electromagnetic fields in the vicinity of heart 24. The generated fields are sensed by a position sensor 32 inside the distal end of catheter 28.

Position sensor 32 is one type of position transducer. In an alternative embodiment, the position transducer in the distal end of catheter 28 may be configured as a radiator, which generates magnetic fields. In this case, coils 30 may sense these fields. Further alternatively, other types of position transducers may be used in the catheter, such as electrical impedance-based transducers (transmitters or sensors), as are known in the art.

The position tracking subsystem in system 20 may operate on the principles of magnetic position tracking systems that are known in the art. Systems of this sort are described, for example, in U.S. Patents 6,690,963, 6,618,612 and 6,332,089, and U.S. Patent Application Publications 2002/0065455 A1, 2004/0147920 A1 and 2004/0068178 A1, whose disclosures are all incorporated herein by reference. A tracking system of this type is used in the CARTO^{™} system, which is produced by Biosense Webster Inc. (Diamond Bar, California). Alternatively, as noted above, the principles of the present invention may be implemented, *mutatis mutandis*, using any other suitable positioning subsystem.

Catheter 28 also comprises an ultrasonic transducer 34 at its distal end, for use in creating images of heart 24. Typically, transducer 34 captures multiple intracardiac ultrasound images (which may be two-dimensional or three-dimensional images). The coordinate readings provided by position sensor 32 are used in registering the ultrasound images captured at different positions of the catheter in order to reconstruct a full 3D image. This image may comprise one or more chambers of the heart, as well as nearby structures outside the heart, such as blood vessels. A catheter and system with such capabilities (and also including an electrode for electro-anatomical sensing) are described in the above-mentioned U.S. Patent Application Publication 2006/0241445.

Further additionally or alternatively, catheter 28 and system 20 may be adapted to create images of other types, such as maps showing mechanical activity or other types of physiological activity within the heart. Furthermore, although the embodiments described herein relate specifically to cardiac imaging, the principles of the present invention may similarly be applied in imaging of other organs of the body.

A console 42 drives and controls the elements of system 20. Console 42 comprises position tracking circuitry 46, which generates signals to drive radiator coils 30 and processes the position signals that are output by position sensor 32 in catheter 28. The signals from the position sensor are conveyed from the catheter via a cable 40 to isolation circuitry 48, which couples the position sensor to position tracking circuitry 46 without creating a conductive path between the sensor and the position tracking circuitry. Thus, catheter 28 itself is isolated from the position tracking circuitry. Details of the isolation circuitry are shown in the figures that follow.

Image signals output by ultrasonic transducer 34 are conveyed from catheter 28 via a cable 38 to image processing circuitry 44. This circuitry processes the image signals in order to generate a 3D ultrasound image of heart 24, as noted above. The image is presented on an output device, such as a display 50. Typically, circuitry 44 comprises a general-purpose computer, with suitable interface circuits and possibly hardware acceleration circuits. The computer is programmed in software to combine the individual images into the 3D image. This process, using the position coordinates computed by position tracking circuitry 46, is described in greater detail in the patents and patent applications cited above.

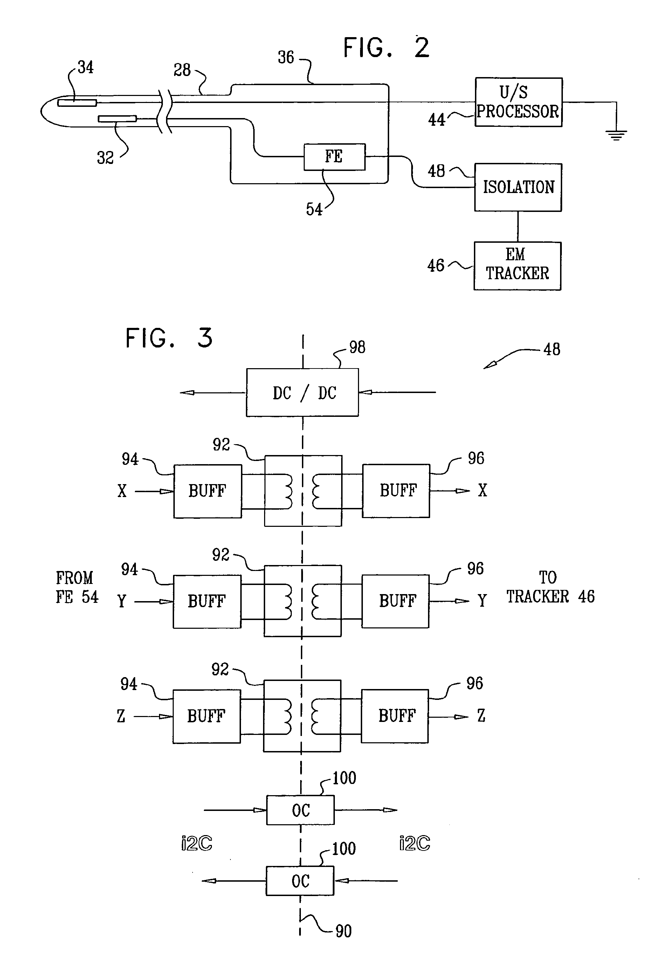

Fig. 2 is a block diagram showing electrical components of system 20, in accordance with an embodiment of the present invention. Position signals output by sensor 32 are amplified by front end (FE) circuitry 54 in handle 36 of catheter 28. The amplified signals pass through isolation circuitry 48 to electromagnetic (EM) position tracking circuitry 46. Circuitry 46 is thus isolated from ultrasonic (U/S) image processing circuitry 44, which is.grounded. Details of isolation circuitry 48 are shown in Fig. 3. Catheter 28 may also contain means for isolating ultrasonic transducer 34 and its associated cabling from position sensor 32, but these features of the catheter are beyond the scope of the present invention.

Fig. 3 is a block diagram showing details of isolation circuitry 48, in accordance with an embodiment of the present invention. Circuitry 48 comprises an isolation barrier 90, made up of suitable isolation components for breaking the conductive path between catheter 28 and position tracking circuitry 46. Position signals from front end circuitry 54 pass through isolation transformers 92 before reaching tracking circuitry 46. Any suitable isolation transformers may be used for this purpose, such as model SM-502-1 transformers distributed by Datatronics (Romoland, California). The three signals (X, Y, Z) originate from three mutually-orthogonal sensor coils making up sensor 32 in catheter 28. (Alternatively, a smaller or larger number of sensor coils may be used, and hence a smaller or larger number of signals may be conveyed by the isolation circuitry.) The sensor signals are buffered at the input to and output from the transformers by buffer amplifiers 94 and 96, such LT6011 amplifiers distributed by Linear Technology (Milpitas, California).

Isolation barrier 90 may also comprise ancillary components, such as an isolated DC/DC converter 98 for providing operating voltage to front end circuitry 54 in handle 36, as well as opto-couplers 100 for transferring digital control and data signals. In the embodiment shown in Fig. 3, the opto-couplers are used to convey two-way communications over an i2C bus, but any other suitable type of communications bus and protocol may be used.

Thus, patient 22 is protected from leakage currents and high-voltage transients that might otherwise penetrate through to heart 24 via catheter 28. Although Fig. 3 shows a certain specific configuration of the isolation circuits, the principles of isolation between different subsystems associated with an invasive probe that are described hereinabove may similarly be applied using other circuits and means of isolation and in systems of other types. It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Medical apparatus, comprising:
an invasive probe, for insertion into a body of a living subject;
an ultrasonic subsystem, comprising an ultrasonic transducer contained in the probe and image processing circuitry, which is located outside the probe and is coupled to communicate with the ultrasonic transducer in the probe; and
a position sensing subsystem, comprising a position transducer contained in the probe and position tracking circuitry, which is located outside the probe and is coupled to communicate with the position transducer so as to determine position coordinates of the ultrasonic transducer in the body,
wherein the position sensing subsystem is electrically isolated from the ultrasonic subsystem.

2. The apparatus according to claim 1, wherein the invasive probe comprises a catheter for insertion into a heart of the subject.

3. The apparatus according to claim 1, wherein the ultrasonic transducer is configured to capture a plurality of ultrasonic input images of an organ at different, respective positions of the probe within the body, and wherein the image processing circuitry is configured to combine the input images, using the position coordinates, to generate a three-dimensional image of the organ.

4. The apparatus according to claim 1, wherein the position sensing subsystem comprises one or more magnetic field generators, which are configured to generate a magnetic field within the body, and wherein the position transducer comprises a magnetic field sensor, which is configured to output position signals to the position tracking circuitry responsively to the magnetic field.

5. The apparatus according to claim 1, wherein the ultrasonic transducer is held at a ground potential by the ultrasonic subsystem, while the position sensing subsystem is at a non-ground potential.

6. The apparatus according to claim 1, and comprising isolation circuitry, which is interposed between the position transducer and the position tracking circuitry so as to convey position signals between the position transducer and the position tracking circuitry without creating a conductive path between the position transducer and the position tracking circuitry.

7. The apparatus according to claim 6, wherein the isolation circuitry comprises one or more isolation transformers.

8. A method for producing an invasive medical system, comprising:
providing an invasive probe, for insertion into a body of a living subject;
assembling an ultrasonic subsystem by installing an ultrasonic transducer in the probe and coupling the ultrasonic transducer to communicate with image processing circuitry outside the probe;
assembling a position sensing subsystem by installing a position transducer in the probe and coupling the position transducer to communicate with position tracking circuitry outside the probe so as to determine position coordinates of the ultrasonic transducer in the body; and
electrically isolating the position sensing subsystem from the ultrasonic subsystem.

9. The method according to claim 8, wherein the invasive probe comprises a catheter for insertion into a heart of the subject.

10. The method according to claim 8, wherein the ultrasonic transducer is configured to capture a plurality of ultrasonic input images of an organ at different, respective positions of the probe within the body, and wherein the image processing circuitry is configured to combine the input images, using the position coordinates, to generate a three-dimensional image of the organ.

11. The method according to claim 8, wherein the position sensing subsystem comprises one or more magnetic field generators, which are configured to generate a magnetic field within the body, and wherein the position transducer comprises a magnetic field sensor, which is configured to output position signals to the position tracking circuitry responsively to the magnetic field.

12. The method according to claim 8, wherein assembling the ultrasonic subsystem comprises holding the ultrasonic transducer, while the position sensing subsystem is at a non-ground potential.

13. The method according to claim 8, wherein electrically isolating the position sensing subsystem comprises interposing isolation circuitry between the position transducer and the position tracking circuitry so as to convey position signals between the position transducer and the position tracking circuitry without creating a conductive path between the position transducer and the position tracking circuitry.

14. The method according to claim 13, wherein the isolation circuitry comprises one or more isolation transformers.
